# EUROPEAN PATENT APPLICATION

(11) **EP 1 417 964 A1**
(43) Date of publication of application: **12.05.2004**
(21) Application number: 02749307.1
(22) Date of filing: 17.07.2002
(51) Int. Cl.: A61K 31/335, A61K 31/765, A61P 35/00, C07D 323/00

(54) **ANTITUMOR AGENT CONTAINING CYCLIC POLYLACTIC ACID**

(30) Priority: 18.07.2001 JP 2001217719
(71) Applicant: AMATO PHARMACEUTICAL PRODUCTS, LTD., Fukuchiyama-shi, Kyoto 620-0932 (JP)
(72) Inventor: WATANABE, Mikio, Hadano-shi, Kanagawa 257-0002 (JP); NAGATO, Yasukazu, Atsugi-shi, Kanagawa 243-0122 (JP); MURAYAMA, Chieko, Ebina-shi, Kanagawa 243-0431 (JP); MURAKAMI, Masahiro, Hirano-ku, Osaka-shi, Osaka 547-0026 (JP); TAKADA, Shigeo, Isehara-shi, Kanagawa 259-1112 (JP)
(74) Representative: Sternagel, Fleischer, Godemeyer & Partner Patentanwälte
(86) International application number: PCT/JP2002/007258
(87) International publication number: WO 2003/007937

(57) **Abstract**

An object of the present invention is to provide a novel antitumor agent by evaluating the antitumor effect which is expressed by a mixture of polylactic acids which contains cyclic polylactic acids as main component. The present invention provides an antitumor agent which comprises a mixture of polylactic acids which contains cyclic polylactic acids having a condensation degree ranging from 3 to 20 as main component.

## Description

### Technical Field

The present invention relates to an antitumor agent, more specifically, an antitumor agent comprising a mixture of polylactic acids which contains cyclic polylactic acids as main component. The antitumor agent according to the present invention shows an effect of reducing a tumor size and an effect of suppressing metastasis; and is useful for treatment of cancer.

### Background Art

Studies on that a mixture of polylactic acid containing cyclic polylactic acid inhibits anaerobic glycolysis system of a cancer cell and expresses an antitumor effect has been conducted mainly with respect to an effect of inhibiting carcinogenesis by using a mouse having a spontaneously generated cancer and an effect of suppressing tumor propagation and metastasis by using a transplanting cancer tissue (Lewis lung cancer cell) (Nagato et al., 56th annual meeting, Japan Cancer Society, 1997, September. and Takada et al., 57th annual meeting, Japan Cancer Society, 1998, September).

The mixture of polylactic acid has been further studied for a chemoprevention effect, as well as an effect of a combined use with an anticancer agent and radioactive irradiation, a n administration method and an administration dose. An experiment of administering a high dose of the agent showed no prominent antitumor effect. In addition, the mixture of polylactic acid which has been so far used contains other substance (e. g., a linear polylactic acid) than the cyclic substance. Namely, it is clear that the mixture of polylactic acid conventionally used contains a cyclic substance and a linear substance, which have different degree of polymerization (also referred to as condensation degree). A proportion of the cyclic substance and the linear substance and an oligomer having a specific degree of polymerization (condensation degree) have not been quantified. In consideration of these situations, it has been attempted to selectively synthesize only the cyclic polylactic acid.

### Disclosure of the Invention

A problem to be solved by the present invention is to provide a novel antitumor agent by evaluating the antitumor effect which is expressed by a mixture of polylactic acids which contains cyclic polylactic acids as main component. Another problem to be solved by the present invention is to provide food and drink prepared by using the antitumor agent as described above.

The present inventors have intensively studied to solve the problems as described above. As a result, they have conducted an experiment where an antitumor effect of a mixture of polylactic acids which contains cyclic polylactic acids as main component (named AD-3 in the following examples) was compared with that of a mixture of polylactic acids which contains a cyclic substance and a linear substance (named CPL in the following examples) which were prepared by a conventional method, and have succeeded in confirming the utility of the former mixture. Particularly, it has been found that the mixture of polylactic acids which contains cyclic polylactic acids as main component, shows a sufficient antitumor effect in a lower dose. The present invention has been completed on the basis of these findings.

According to the invention, there is provided an antitumor agent which comprises a mixture of polylactic acids which contains cyclic polylactic acids having a condensation degree ranging from 3 to 20 as main component.

Preferably, the mixture of polylactic acids used in the invention contains substantially no linear polylactic acid.

Preferably, the mixture of polylactic acids which contains cyclic polylactic acids having a condensation degree ranging from 3 to 20 as main component, is prepared by a method of preparation which comprises:
(i) a first heating step wherein lactic acid is subjected to dehydration and condensation by heating, and condensation and dehydration is carried out under pressure and temperature conditions such that byproduct water is distilled and removed while distillation of lactide is avoided; and
(ii) a second heating step wherein at a temperature higher than that of the first step, the pressure is reduced to 100 mmHg or lower under the pressure and temperature condition such that byproduct water is distilled and removed while distillation of lactide is avoided, and a reaction product of the first heating step is subjected to a further continuing reaction at the reduced pressure under heating so as to produce a dehydrated and condensed product of lactic acid.

According to another aspect of the present invention, there are provided food and drink which comprise the antitumor agent according to the invention as described above.

According to further aspect of the present invention, there is provided a use of a mixture of polylactic acids which contains cyclic polylactic acids having a condensation degree ranging from 3 to 20 as main component, in the production of the antitumor agent or the food and drink for an antitumor effect.

According to still further aspect of the present invention, there is provided a method for suppressing tumor which comprises administering an effective amount of a mixture of polylactic acids which contains cyclic polylactic acids having a condensation degree ranging from 3 to 20 as main component, to mammal animals including human.

### Brief Description of the Drawings

Fig. 1 shows a mass spectrum of the mixture of polylactic acids obtained in Synthesis Example 1.
Fig. 2 shows an MS spectrum of a reaction product obtained in Synthesis Example 2.
Fig. 3 shows a whole view of an NMR of a reaction product obtained in Synthesis Example 2.
Fig. 4 shows an enlarged view of a part of Fig. 3.
Fig. 5 shows an enlarged view of a part of Fig. 3.
Fig. 6 shows a comparison of weights of tumor tissues in Example 1.
Fig. 7 shows a weight of the tumor tissue (parenchymal tissue and spongy tissue) of a group received the AD-3 in Example 1.
Fig. 8 shows the comparison of numbers of colony metastasized to a lung in the group received the AD-3 in Example 1.
Fig. 9 shows the comparison of proportions of tumor tissues in Example 2.
Fig. 10 shows the comparison of individual tissue weights in Example 2.

### Best Mode for carrying out the Invention

The embodiment and method for the practice of the present invention are described in detail below.

In the antitumor agent and the food and drink for antitumor effect according to the present invention, a mixture of polylactic acids which contains cyclic polylactic acids having a condensation degree ranging from 3 to 20 as main component, is used as an active ingredient.

The term "a mixture of polylactic acids" used in the present invention means a mixture wherein cyclic poly lactic acids having a condensation degree of 3 to 20 are present at any ratio. Although the term "a mixture of polylactic acids" is used in the present specification for the sake of convenience, this term also includes a poly lactic acid consisting of a single ingredient such as a cyclic poly lactic acid having single condensation degree.

The term "condensation degree" is used to mean the number of lactic acid unit that is a repeating unit in poly lactic acids. For example, the cyclic poly lactic acid is assumed to have the following structural formula. In the formula, when m is 1, the condensation degree is 3; and when m is 18, the condensation degree is 20. 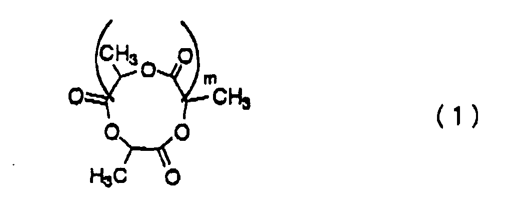 wherein m represents an integer of 1 to 18.

When "lactic acid" is simply referred to in the present specification, this lactic acid includes all of L-lactic acid, D-lactic acid or a mixture comprising these types of lactic acid at any ratio. Preferably in the present invention, the lactic acid consists substantially of L-lactic acid. The term "substantially" is used herein to mean that the ratio of L-lactic acid units in a mixture of poly lactic acids (number of L-lactic acid unit / number of L-lactic acid unit + number of D-lactic acid unit x 100) is, for example, 70% or more, preferably 80% or more, more preferably 85% or more, further more preferably 90% or more, and particularly preferably 95% or more. The ratio of L-lactic acid units in a mixture of poly lactic acids depends on the ratio of L-lactic acid and D-lactic acid that exist in lactic acids used as a starting substance.

The antitumor agent according to the invention can be widely used for suppressing a tumor. Suppressing the tumor includes prevention of oncogenesis, suppression of tumor enlargement, regression of tumor, and suppression of tumor metastasis, and also includes clinically all of prevention and/or treatment of a cancer and/or a tumor.

The type of the cancer for which the antitumor agent according to the invention can be used is not particularly limited, but includes all of benign tumors and malignant tumors. Examples of the cancers include malignant melanoma, malignant lymphoma, digestive tract cancers, lung cancer, esophagus cancer, stomach cancer, large bowel cancer, rectum cancer, colonic cancer, urinary tract tumor, gallbladder cancer, cholangiocarcinoma, biliary tract cancer, breast carcinoma, hepatic cancer, pancreatic cancer, testis tumor, cancer of the upper jaw, cancer of the tongue, cancer of the lip, cancer of the oral cavity, cancer of pharynx, cancer of larynx, ovarian cancer, cancer of the uterus, cancer of prostate, cancer of thyroid, brain tumor, Kaposi's sarcoma, angioma, leukemia, plethora vera, neuroblastoma, glioma retinae, myeloma, bladder tumor, sarcoma, osteogenic sarcoma, myosarcoma, cancer of skin, basal cell cancer, skin appendage carcinoma, skin metastatic cancer, skin melanoma and, however, is not limited to these examples.

### (A) A method of preparing the mixture of polylactic acid used in the present invention

The antitumor agent according to the invention contains, as an active ingredient, a mixture of polylactic acids which contains cyclic polylactic acids having a condensation degree ranging from 3 to 20 as main component.

### (A-1) A first method of preparation

A first method of preparing a mixture of polylactic acids used in the present invention comprises :
(i) a first heating step wherein lactic acid is subjected to dehydration and condensation by heating, and condensation and dehydration is carried out under pressure and temperature conditions such that byproduct water is distilled and removed while distillation of lactide is avoided; and
(ii) a second heating step wherein at a temperature higher than that of the first step, the pressure is reduced to 100 mmHg or lower under the pressure and temperature condition such that byproduct water is distilled and removed while distillation of lactide is avoided, and a reaction product of the first heating step is subjected to a further continuing reaction at the reduced pressure under heating so as to produce a dehydrated and condensed product of lactic acid.

The starting material, lactic acid, used in the invention may include any one of D-lactic acid, L-lactic acid, or DL-lactic acid. One of these lactic acids may be singly used or a combination of two or more lactic acids may be used.

The first heating step is a step of condensation by dehydration by heating lactic acid, which is the step of condensation by dehydration under the pressure and temperature condition to enable distillation and removal of byproduct water, while avoiding distillation of lactide. The reaction of this first heating step leads distillation and removal of byproduct water, which is produced by condensation by dehydration of lactic acid, in order to make the reaction go smoothly. In such the case, the pressure and temperature are set to prevent distillation and removal of lactide, which is the product of condensation by dehydration of two molecules of lactic acid.

The pressure for the reaction may be a normal pressure or a reduced pressure, preferably a reduced pressure. The pressure for the reaction ranges specifically from 10 to 760 mmHg, preferably 300 to 500 mmHg, and more preferably 350 to 400 mmHg. The temperature for the reaction, depending on the pressure condition, normally ranges from 100 °C to 150 °C and more preferably 120 °C to 140 °C.

The period for the reaction of this first heating step is not specially limited, and normally ranges from 3 to 12 hours and preferably 5 to 6 hours.

By the reaction of this first heating step, a reaction product is produced having the product of condensation by dehydration of lactic acid of 3 to 23 molecules as the main component.

After completion of the first heating step as described above, in the second heating step, in order to yield an oligomer having a higher average degree of polymerization, heating is carried out to increase the temperature to a higher temperature than the reaction temperature in the first heating step as described above, e. g., 145°C or higher, preferably 150°C to 180°C, and more preferably 150°C to 160°C, while reducing the reaction pressure to 100 mmHg or lower, preferably 10 to 80 mmHg, and more preferably 15 to 20 mmHg to continue dehydration and condensation reaction.

In this reaction, similar to the reaction of the first heating step as described above, byproduct water is removed to make the reaction go smoothly, and the reaction is carried out in a condition to avoid removal of lactide by distillation.

The rate for reducing the reaction pressure (pressure reducing rate) to the pressure within the above-descried range (100 mmHg or lower) is necessary to be kept to the rate lower than 5 mmHg/min to prevent removal of lactide by distillation and increase reaction efficiency. The rate for reducing the reaction pressure is preferably 0.25 mmHg/min or higher and lower than 5 mmHg/min, more preferably 0.25 mmHg/min or higher and 4 mmHg/min or lower, further preferably 0.5 mmHg/min or higher and 3 mmHg/min or lower, and particularly preferably 0.5 mmHg/min or higher and 1 mmHg/min or lower. The rate for reducing the pressure lower than the range as described above is not preferable, because a longer time is required for reducing the pressure to the predetermined pressure, and also the rate of 5 mmHg/min or higher for reducing the pressure is not preferable, because lactide is removed by distillation together with byproduct water.

Following the reaction pressure reduced to the pressure of 100 mmHg or lower, the reaction is continued at this reaction pressure. On this step, the reaction period ranges from 3 to 12 hours, preferably from 5 to 6 hours.

The reaction in the second heating step yields a lactic acid oligomer having an average degree of polymerization ranging from 3 to 30, preferably from 3 to 23. The proportion of the cyclic lactic acid oligomer to all lactic acid oligomers contained in the reaction product of the second heating step is normally 70% by weight or more and, for example, ranges from 70 to 80% by weight.

Preferably, following the second heating step as described above, a third heating step is operated. The third heating step is a step of producing the cyclic lactic acid oligomer by heating the reaction product of the second heating step at a pressure lower than that for the second heating step so as to allow the linear lactic acid oligomer contained in the reaction product to be cyclized.

The reaction pressure for the third heating step ranges preferably from 0.1 to 5 mmHg, more preferably from 0.25 to 5 mmHg, further preferably from 0.5 to 3 mmHg, and particularly preferably from 0.5 mmHg to 1 mmHg. The temperature for the reaction for the third heating step ranges preferably from 145 to 180°C and more preferably 150 to 160°C.

The reaction is continued at such pressure and temperature conditions. The reaction period ranges from 3 to 12 hours, preferably from 5 to 6 hours. In this step, byproduct water is removed by distillation. Preferably in this step, removal of lactide by distillation is avoided. However, the reaction product contains almost no lactide and, hence, the rate for reducing the pressure may not be necessarily decreased.

The reaction in the third heating step yields the cyclic lactic acid oligomer having an average degree of polymerization ranging from 3 to 30 and preferably from 3 to 23. The proportion of the cyclic lactic acid oligomer to all lactic acid oligomers contained in the reaction product of the third heating step is normally 90 % by weight or more and, preferably, ranges 99 % by weight or more.

### (A-2) Second preparation method

A second method for preparation of a mixture of polylactic acids used in the invention includes a method of polymerizing lactide in the presence of an alkali metal compound represented by the following formula (2):

R-Y-Me (2)

wherein R represents an aliphatic group, an aromatic group, -Si(R¹⁰)(R¹¹)(R¹²), -CH(R²⁰)CONR²¹R²², or -CH(R³⁰)COOR³¹, wherein R¹⁰, R¹¹ and R¹² represent independently an aliphatic group or an aromatic group, R²⁰ represents an aliphatic group, R²¹ and R²² represent independently a hydrogen atom, an aliphatic group, or an aromatic group, and R³⁰ represents an aliphatic group, and R³¹ represents a hydrogen atom, an aliphatic group or an aromatic group;
Y represents -O-, -S-, or -NR⁴⁰-, wherein R⁴⁰ represents a hydrogen atom, an aliphatic group or an aromatic group; and
Me represents an alkali metal.
Lactide (3,6-dimethyl-1,4-dioxane-2,5-dione) which is made by dehydration condensation of two molecules of lactic acids is used as a starting material. This lactides are reacted in the presence of the alkali metal compound represented by the formula (2) as described above. The formula (2):

R-Y-Me (2)

will be described below.

In the formula (2), R represents an aliphatic group, an aromatic group, -Si(R¹⁰)(R¹¹)(R¹²), -CH(R²⁰)CONR²¹R²², or -CH(R³⁰)COOR³¹, wherein R¹⁰, R¹¹ and R¹² represent independently an aliphatic group or an aromatic group, R²⁰ represents an aliphatic group, R²¹ and R²² represent independently a hydrogen atom, an aliphatic group or an aromatic group, R³⁰ represents an aliphatic group, and R³¹ represents a hydrogen atom, an aliphatic group or an aromatic group.

The aliphatic group in the invention includes saturated or unsaturated aliphatic hydrocarbon groups having 1 to 12, preferably 1 to 6 carbon numbers, which may be linear, branched, cyclic or a combination thereof. Examples thereof include an alkyl group such as methyl, ethyl, n-propyl, i-propyl, n-butyl, i-butyl, t-butyl, octyl and dodecyl; a cycloalkyl group such as cyclopropyl, cyclobutyl, cyclooctyl and cyclododecyl. The aliphatic group may be an unsaturated hydrocarbon group having a double bond or a triple bond.

The aromatic group in the invention includes an aryl or aryl alkyl group having 6 to 30, preferably 6 to 20, more preferably 6 to 12, and further preferably 6 to 10 carbon numbers. Examples of the aryl group include phenyl, tolyl, and naphthyl, and examples of the aryl alkyl group include benzyl, phenethyl, and naphthylmethyl.

The aliphatic group and the aromatic group may have 1 or more substituents. The types of the substituent is not specially restricted, but includes a straight chained or branched, linear or cyclic alkyl group, a straight chained or branched, linear or cyclic alkenyl group, a straight chained or branched, linear or cyclic alkinyl group, an aryl group, an acyloxy group, an alkoxy carbonyloxy group, an aryloxycarbonyloxy group, a carbamoyloxy group, a carbonamide group, a sulfonamide group, a carbamoyl group, a sulfamoyl group, an alkoxy group, an aryloxy group, an aryloxycarbonyl group, an alkoxycarbonyl group, an N-acylsulfamoyl group, an N-sulfamoylcarbamoyl group, an alkylsulfonyl group, an arylsulfonyl group, an alkoxycarbonylamino group, an aryloxycarbonylamino group, an amino group, an ammonio group, a cyano group, a nitro group, a carboxyl group, a hydroxyl group, a sulfo group, a mercapto group, an alkylsulfynyl group, an arylsulfynyl group, an alkylthio group, an arylthio group, an ureido group, a heterocyclic group such as a 3- to 12-member single ring and condensed ring having at least 1 or more nitrogen, oxygen, sulfur or the like, a heterocyclic oxy group, a heterocyclic thio group, an acyl group, a sulfamoylamino group, a silyl group, a halogen atom, and the like. Of those described above, the carbon number of the alkyl group, alkenyl group, alkinyl group and alkoxy group ranges generally from 1 to 12, preferably from 1 to 6 and that of the aryl group ranges generally from 6 to 20, preferably from 6 to 10.

In the formula (2), Y represents -O-, -S- or -NR⁴⁰-, wherein R⁴⁰ represents a hydrogen atom, an aliphatic group or an aromatic group. Preferably, Y represents -O- or -S-. The aliphatic group or the aromatic group represented by R⁴⁰ is as described above.

In the formula (2), Me represents an alkali metal. The alkali metal includes, for example, Li, Na or K, preferably Li.

A compound represented by the formula (2) and having an asymmetric carbon atom may be any of (R) form, (S) form or (R), (S) form.

A method for obtaining the alkali metal compound represented by the formula (2) is not specially restricted, and those skilled in the art can suitably obtain it by reacting an alkylated alkali metal such as n-butyl lithium with R-YH.

When lactide is polymerized in the presence of the alkali metal compound represented by the formula (2) according to the process of the present invention, the amount of the alkali metal compound (R-Y-Me) is preferably 1 to 0.1 mole, more preferably 0.2 to 0.3 mole, per 1 mole of lactide.

The reaction temperature of the aforementioned reaction is not specially restricted as long as the reaction goes well, but preferably ranges from -100 °C to a room temperature, more preferably ranges from -78°C to -50°C. The reaction is preferably conducted by starting at a temperature ranging from -78°C to -50°C and raising the temperature gradually to the room temperature.

The reaction of polymerizing lactide in the method as described above is conducted preferably in the presence of a reaction solvent. The reaction solvent is not specially restricted as long as the solvent is inert to the reaction. Preferably, a cyclic ether such as tetrahydrofuran, diethyl ether, dimethoxy ethane or the like can be used. The reaction atmosphere includes an atmosphere of an inert gas such as nitrogen gas, argon gas and the like. The pressure for the reaction is not specially restricted, but preferably the normal pressure.

The composition of the lactic acid oligomer which is yielded by the method as described above varies according to the alkali metal compound used as a reaction aid. Using an alkali metal compound (preferably a lithium compound) of an alkyl alcohol having 1 to 3 carbon numbers, for example, as the alkali metal compound, yields a mixture of the cyclic lactic acid oligomer with the linear oligomer (proportion of the cyclic lactic acid oligomer ranges from 80 to 85 % by weight). On the other hand, using the alkali metal compound of the alkyl alcohol such as t-butyl alcohol, which has 4 or higher carbon numbers, or the alkali metal compound of thiophenol and the like as an alkali metal compound can yield substantially selectively only the cyclic lactic acid oligomer. Alternatively, using a compound in which R in the formula (2) represents -CH(R²⁰)CONR²¹R²² where R²⁰ represents an aliphatic group, and R²¹ and R²² represent independently a hydrogen atom, an aliphatic group or an aromatic group, more specifically, for example, a lactic acid amide represented by the following formula (3), as the alkali metal compound, can yield substantially selectively only the cyclic lactic acid oligomer. 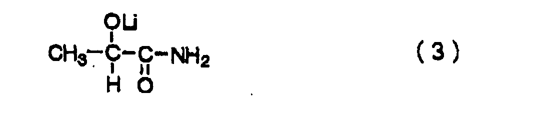 "can yield substantially selectively only the cyclic lactic acid oligomer" expressed in this specification means that no linear lactic acid oligomer is substantially produced in the reaction product. It means specifically that a ratio of the linear lactic acid oligomer to all lactic acid oligomers in the reaction product is generally 10% by weight or lower, preferably 5% by weight or lower, and particularly preferably 3% by weight or lower.

The cyclic lactic acid oligomer produced by the first method and the second method as described above is expected to have the following chemical structure. 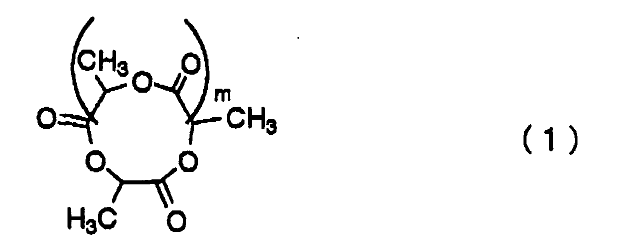 wherein m represents an integer ranging from 1 to 30.

The reaction product according to the method as described above is the mixture of the cyclic lactic acid oligomer showing normally m which is the integer ranging from 1 to 30, for example, 1 to 28, 1 to 25, 1 to 21, 1 to 18, or the like.

According to a preferable embodiment, substantially without any production of the linear lactic acid oligomer, the cyclic lactic acid oligomer can be selectively produced. Hereby, "substantially without any production of the linear lactic acid oligomer" means that the ratio of the cyclic lactic acid oligomer to all lactic acid oligomers in the reaction product is 80% by weight or more, preferably 90% by weight or more, more preferably 95% by weight or more, and particularly preferably 99% by weight or more.

The antitumor agent according to the present invention comprises, as an active ingredient, the mixture of polylactic acids which contains the cyclic polylactic acids, which can be produced according to the preparation method as described above.

It is preferable that the mixture of polylactic acids used in the present invention contains substantially no linear polylactic acid. Hereby, "contains substantially no" means that the ratio of the linear polylactic acid to all polylactic acids is 20% by weight or lower, preferably 10% by weight or lower, more preferably 5% by weight or lower, and particularly preferably 1% by weight or lower.

### (B) Method for preparation and method for use of the antitumor agent according to the present invention

The antitumor agent according to the present invention can also be used in combination with other antitumor agents and/or immunotherapic agents. Other antitumor agent includes mitomycin, adriamycin, cisplatin, vindesine, vincristine, cyclophosphamide, ifosfamide, bleomycin, peplomycin, or etoposide. On the other hand, other immunotherapic agents include a microorganism or cell wall skeleton component of a bacterium, an immunoreactive polysaccharide, a cytokine being natural type or obtained by a genetic engineering approach, or a colony-stimulating factor. The immunoreactive polysaccharide as described above includes lenthinan, schizophyllan and the like, the bacterial cell wall skeleton component includes muramyl dipeptide derivatives and the like, the microorganism includes a lactic acid bacterium and the like, and the cytokine being the natural type or obtained by the genetic engineering approach includes an interferon and the like.

When required, the antitumor agent according to the present invention can be added to the components as described above with components or additives used for preparation of a dug such as medical drugs, quasi-drugs and the like by free selection and combination in a range which does not damage an effect of the invention. The antitumor agent according to the present invention can be used by compounding it in medical drugs, quasi-drugs and the like in addition to a use as a single drug.

The form of the antitumor agent according to the invention is not specially restricted, but the appropriate form most suitable for a purpose can be selected from drug forms for oral administration or parenteral administration.

The drug preparation form suitable for oral administration includes, for example, a tablet, capsule, powder, drink, granule, fine granule, syrup, solution, emulsion, suspension, chewable and the like. The drug preparation form suitable for parenteral administration includes, for example, injection (subcutaneous injection, intramuscular injection, intravenous injection and the like), external use, drip, inhalation, spray and the like and, however, is not restricted to these forms.

A liquid drug preparation, for example, solution, emulsion or syrup, which is suitable for oral administration, can be prepared by using water, saccharides such as sucrose, sorbit and fructose, glycols such as polyethylene glycol and propylene glycol, oils such as sesame oil, olive oil and soybean oil, antiseptic agents such as p-hydroxy benzoic acid esters, and flavors such as strawberry flavor and peppermint. On the other hand, solid drug preparation, for example, tablet, capsule, powder, granule and the like, can be prepared by using an excipient such as lactose, glucose, sucrose and mannite, a disintegrating agent such as starch and sodium alginate, a lubricant such as magnesium stearate and talc, a binder such as polyvinyl alcohol, hydroxy propyl cellulose and gelatin, a surfactant such as fatty acid ester, a plasticizer such as glycerin.

The drug preparation for injection or drip suitable for parenteral administration includes preferably the material, which is the active ingredient, as described above in a sterilized water-based medium, which is isotonic to blood of a recipient, in a dissolved or suspended condition. For example, in case of the injection, the solution can be prepared by using a water-based medium and the like composed of a salt solution, a glucose solution, or the mixture of the glucose solution with the salt solution. The drug preparation for intestinal administration can be prepared by using a carrier such as cacao butter, hydrogenated fat or hydrogenated carboxylic acid, and can be used as a suppository. In addition, for preparation of spray, a carrier which allows the material being the active ingredient as described above to disperse as fine particles, does not irritate a mouth cavity and an air way mucosa of the recipient, and makes absorption of the active ingredient easy, can be used. The carrier is specifically exemplified by lactic acid, glycerine and the like. In accordance with a property of the material being the active ingredient and the carrier to be used, the drug preparation having forms such as an aerosol or dry powder. These preparations for parenteral administration are also added with 1 or 2 or more species of eatables and drinkables selected from glycols, oils, flavors, antiseptic agents, excipients, disintegrating agents, lubricants, binders, surfactants, plasticizers or the like.

The dose and administration frequency of the antitumor agent according to the present invention can be properly selected in accordance with various factors including a purpose of administration, a form of administration, conditions of a recipient such as an age, body weight and sexuality and, however, as a rule, the amount of administration of the active ingredient ranges from 1 to 10000 mg/kg/day, preferably 10 to 2000 mg/kg/day, more preferably 10 to 200 mg/kg/day. It is preferable to administer the preparation of the amount as described above in 1 to 4 frequencies a day. The time of administration of the antitumor agent according to the present invention is not specially restricted.

The invention also relates to food and drink which comprises the mixture of polylactic acids which contains the cyclic polylactic acids having the condensation degree ranging from 3 to 20 as the main component. Namely, the mixture of cyclic polylactic acids having the condensation degree ranging from 3 to 20 used in the invention can be not only used in the forms of a single preparation as described above, but also used by compounding it in food and drink.

A compounding form of food and drink according to the invention is not specially restricted, when it is satisfied to compound the mixture of polylactic acids without decomposition.

The product of food and drink according to the present invention includes specifically a health food or a supplementary food including beverages, which is generally called a refreshing drink, drink agent, health food, specified health food, functional food, function activating food, nutriceutical food, supplement, feed, feed additive and the like. The antitumor agent according to the present invention can be used as a veterinary drug, feed and the like.

Food and drink include arbitrary food and dinks, and examples thereof include confectionaries such as chewing gum, chocolate, candy, tablet confectionary, jelly, cookie, biscuit and yogurt, cold confectionaries such as ice cream and ice confectionary, beverages such as tea, refreshing drink (including juice, coffee, cocoa and the like), nourishment drink agent and esthetic drink agent, bread, ham, soup, jam, spaghetti, frozen foods. Alternatively, the mixture of cyclic polylactic acids according to the present invention can also be used by adding it to a flavoring material or a food additive. By taking food and drink according to the present invention, the antitumor effect is obtained to provide safe food and drink which show no substantially harmful adverse effect.

The food and drink according to the present invention can be obtained by directly mixing and dispersing the mixture of polylactic acids to a common material used for foods and then processing the same in a desired form by a publicly known method.

The food and drink according to the present invention encompasses food and drink in every form, and the types are not specifically limited. That is, the food and drink can be provided by mixing the antitumor agent of the present invention into the above-mentioned various food and drink, or various nutrient compositions, such as various oral or enteral nutrient preparations or drinks. Compositions of such food and drink may include protein, lipid, carbohydrate, vitamin and/or mineral, in addition to the mixture of cyclic polylactic acids having a condensation degree of 3 to 20. The form of the food and drink is not specifically limited, and may be in any form, such as solid, powdery, liquid, gel, and slurry forms, so far as it is in a form that is easily ingested.

The content of the mixture of poly lactic acids in the food and drink is not specifically limited, and is generally 0.1 to 20 weight %, more preferably approximately 0.1 to 10 weight %.

The mixture of poly lactic acids is preferably contained in the food and drink in an amount which achieve an antitumor effect which is an object of the present invention. Preferably, about 0.1 g to 10 g, more preferably about 0.5 g to 3 g, of the mixture of poly lactic acids is contained per food or drink to be ingested.

The present invention is further described in the following examples, but the scope of the present invention is not limited by the examples in any way.

### EXAMPLES

### Synthesis Example 1: Production of a mixture of poly lactic acids (hereinafter referred to as CPL)

500 ml of L-lactic acid (to which D-lactic acid was also mixed) was placed into a separable flask in a mantle heater. 300ml/min of nitrogen gas was flowed therein while stirring. Accumulated water was introduced into a flask equipped with a reflux condenser via a warmed descending type connecting tube, while heating at 145°C for 3 hours. Furthermore, after pressure was reduced to 150 mmHg and heated at the same temperature for 3 hours, the mixture was heated at 155°C for 3 hours under a reduced pressure of 3 mmHg, and then at 185°C for 1.5 hours under a reduced pressure of 3 mmHg to obtain poly lactic acids as a reaction product.

The obtained poly lactic acids were kept at 100°C, and 100ml of ethanol and 400ml of methanol were separately added thereto, and then the mixture was allowed to be cooled. This mixture was added to 500ml of methanol, and the mixture was well stirred and left to stand. Then, the mixture was filtrated for purification. The filtrate was subjected to vacuum drying and then dissolved in acetonitrile to obtain 200ml (stock solution) in total.

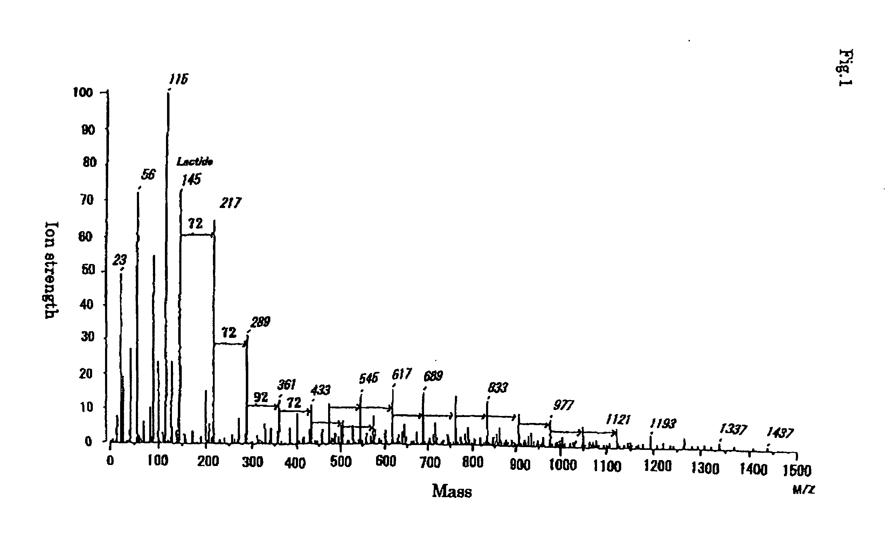

The stock solution was subjected to a reverse phase ODS column (TSK gel ODS-80 TM) which was previously equilibrated, and was stepwise eluted with 30%, 50% and 100% acetonitrile (pH2.0) each containing 0.01M hydrochloric acid to obtain poly lactic acids (condensation degree of 3 to 20) as an acetonitrile 100% elution fraction. The mass spectrum of the obtained substance is shown in Fig. 1. As is clear from the regular fragment ion peaks in Fig. 1, the obtained mixture of poly lactic acids comprises cyclic condensate and a linear condensate in a mixed state.

### Synthesis Example 2: Production of a mixture of cyclic polylactic acids (hereinafter referred to as AD-3)

10.0 g of (s)-(+)-lactic acid was placed in an eggplant-shaped flask having a inner volume of 100 ml, and was loaded on a rotary evaporator. The internal pressure of the flask was regulated to a range from 350 to 400 mmHg, and the mixture was heated to 140°C, followed by continuing the reaction at this temperature and this pressure for 6 hours (first heating step). Byproduct water produced by the reaction was removed by distillation. In the reaction condition as described above, lactide was almost not removed out from the system.

Subsequently, the reaction temperature was increased to 150 to 160°C and the reaction pressure was gradually dropped from 400 mmHg over 6 hours to make finally 15 to 20 mmHg (pressure-dropping rate: 1 mmHg/min). Under the condition of this pressure-dropping rate, byproduct water was removed by distillation and, however, lactide was almost not removed out. Thereafter, the pressure was maintained at 15 to 20 mmHg to continue the reaction for 6 hours (second heating step).

Next, the pressure was dropped to 1 to 3 mmHg over 30 minutes, and the reaction was continued at 160°C for 5 hours (third heating step).

After completion of the reaction as mentioned above, the reaction product was analyzed resulting in the yield of the cyclic oligomer of 6.80 g (yield 85%) having 3 to 21 of average polymerization degree.

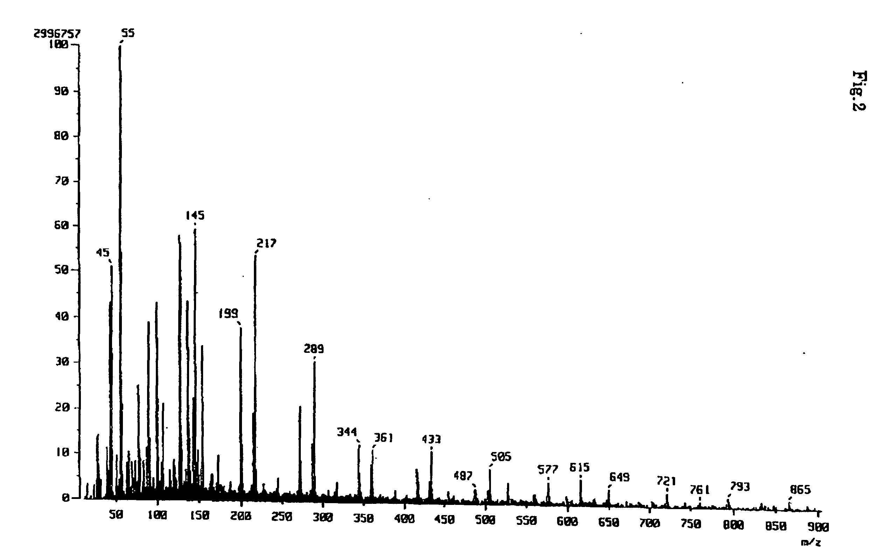

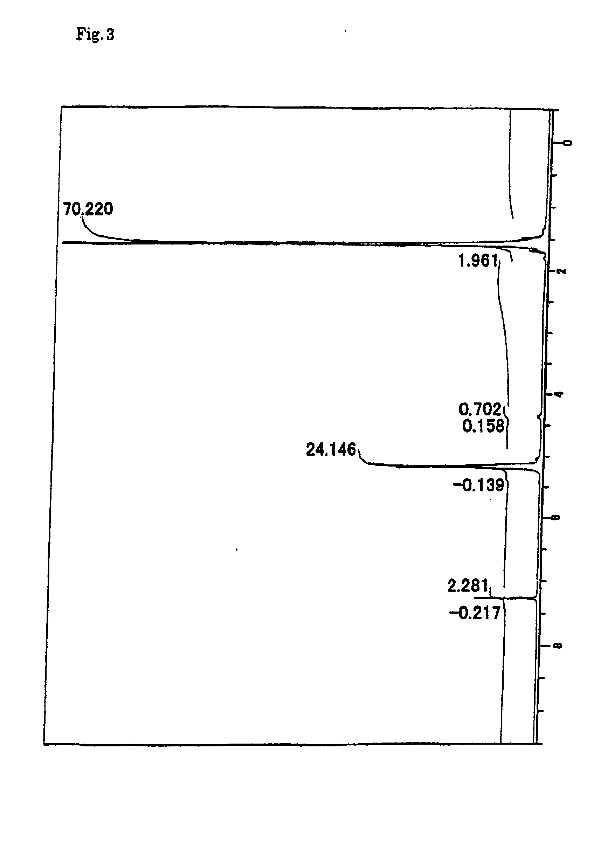

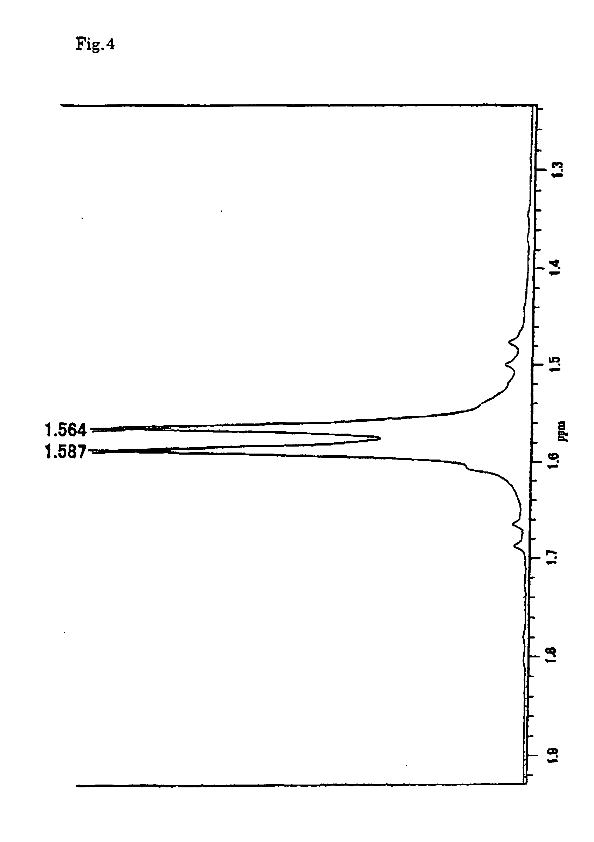

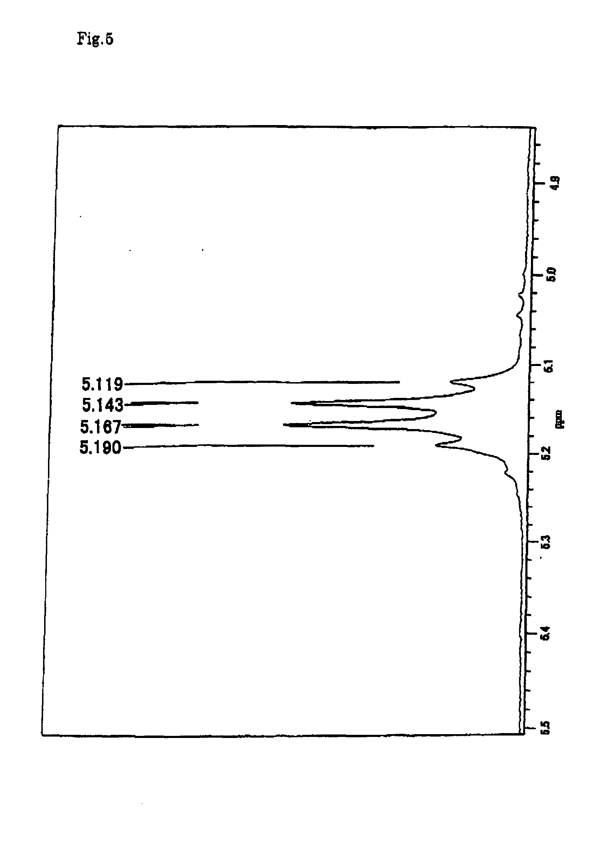

Fig. 2 shows the MS spectrum of the reaction product obtained in Synthesis Example 2. In addition, Fig. 3 shows the whole view of the NMR of the reaction product obtained in Synthesis Example 2. Figs. 4 and 5 show the enlarged views of parts of Fig. 3.

### Example 1:

### (A) Material and method

### (1) Experimental animals and transplantation of tumor cells

1.0 X 10⁴ of Lewis lung cancer cells were transplanted to a right femoral subcutaneous portion of 6-week old mice (C57BL/6N).

### (2) Administration of a test substance

Mice were divided into a control group (the group received a solvent), AD-3-(prepared in the Synthesis Example 2) administered group (the invention), and CPL-(prepared in the Synthesis Example 1) administered group (comparative group). The AD-3- administered group and the CPL- administered group were divided into 4 groups including 0.2 mg-, 0.4 mg-, 1.0 mg-, and 4.0 mg-administering groups, in accordance with the amount of a dose to be administered to each individual.

Started at the 2nd day after transplantation, the solvent received group, AD-3-administered group, and CPL-administered group were subjected to intra-abdominal administration of a solvent (a mixed liquid of glycerin and propylene glycol), AD-3 or CPL dissolved into the solvent, respectively. Until the mouse was euthanized at a period from 17th to 19th days after the administration was started, the administration was conducted on alternate days.

### (3) Collection of transplanting tissue and measurement

After deep anesthesia, thoracotomy was operated to reflux PBS from a left ventricle. Thereafter, the transplanting tumor tissue was resected, measured for a major axis, minor axis, thickness, and weight. In the tumor tissue observed, a tumor cell layer, in which tumor cells aggregated on a surface layer, was found as separated from a spongy tissue, in which a deep portion was filled with blood. Then, after the measurement of the weight, the spongy tissue was removed, and the weight of the tumor cell layer on the surface layer was measured.

### (4) Observation of a colony metastasized to a lung

Following resection of both lungs, metastasized colonies were counted on individual lobes of the lung.

### (5) Microscopical observation of transplanting tissue

A tissue section of the tumor tissue was prepared according to a histological method, and was subjected to microscopic observation after H-E staining.

### (B) Result and discussion

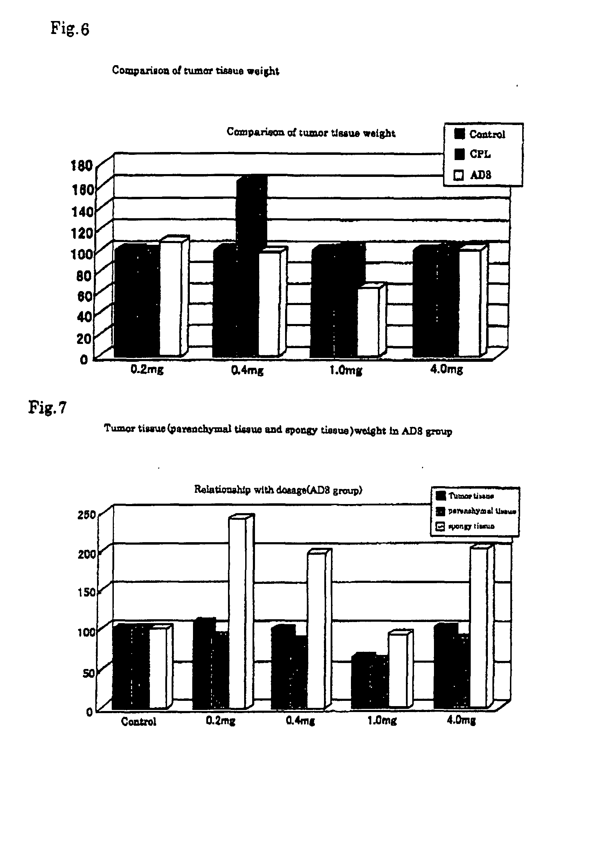

The results are shown in Figs. 6 to 8.

### (1) Tumor tissue

The weight of the tumor tissue of the CPL group was larger in the 0.4 mg administered group than that of the control group and, however, it showed no difference in other administration amount groups. In contrast with this result, the weight of the AD3-administered group reduced to about 65% in the 1.0 mg administered group, showing a distinct tumor suppressing effect compared with the CPL group (Fig. 6 to Fig. 7).

### (2) Observation of tumor tissue

From the result of the tissue section, in the AD3-administered group, infiltration of many neutrophils was found in a boundary region between a parenchymal tissue and the spongy tissue or inside the parenchymal tissue. Neutrophils inside the parenchymal tissue were found around blood capillaries. In addition, infiltration of neutrophils in the AD3 group was found in an overwhelming region in contrast with the CPL group, and the difference was prominent. This infiltration was most prominent on the 1.0 mg administered group in which the most distinct reduction effect was found in the tumor size in the AD3 group, suggesting a relationship to a tumor reduction effect.

### (3) Colony metastasized to lung

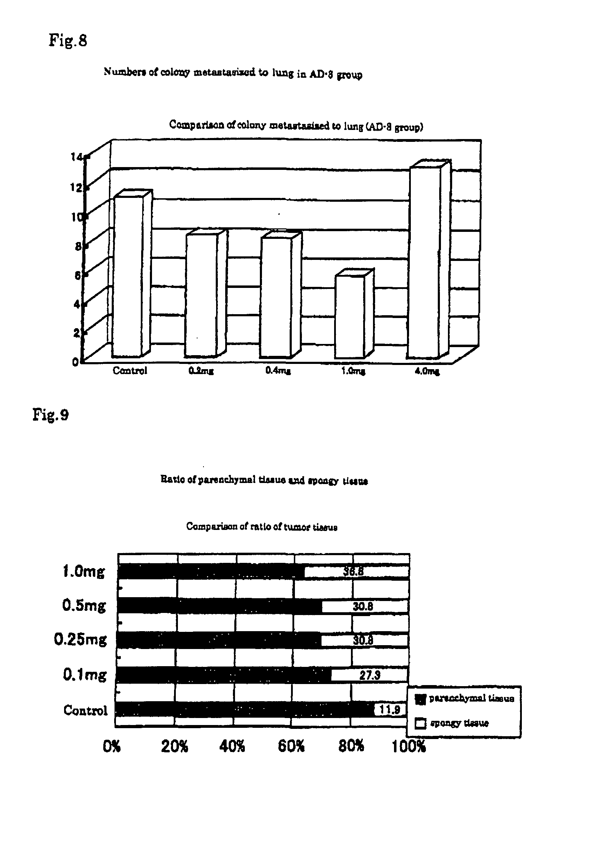

It was found that number of metastasized colonies of the AD3-administered group was smaller than that of the control group and the CPL group. Among them, the 1.0 mg administered group showed about one third of number of colonies in the control group. From these results of observations, it was found that the effect of suppressing propagation and metastasis of transplanting tumor tissue was larger in AD3 being the selective cyclic lactic acid oligomer and the most prominent effect was obtained in the 1.0 mg administered group (Fig. 8).

### (4) Conclusion

A comparison was carries out between the antitumor effect of AD-3 obtained by the method for synthesis of the selective cyclic lactic acid oligomer and that of the CPL prepared by the conventional heating condensation method. The result of examination by changing the administered amount showed the superiority of the tumor reducing effect and the tumor metastasis suppression effect of the AD-3 to those of the CPL.

Administration of the AD-3 caused hematogenous neutrophil infiltration in the tumor tissue. It has been already suggested that a factor of the antitumor effect of the AD-3 and the CPL is based on suppression of an anaerobic glycolytic system. It is suggested that the present result shows that the cyclic lactic acid oligomer has an antitumor effect caused by suppression of an anaerobic glycolytic system, as well as a tumor suppression effect through an immune system.

Among the AD-3-administered groups, the 1.0 mg administered group showed the prominent effect. A difference in optimal concentrations was found, because it has been so far known that intra-abdominal administration of 4 mg of the CPL shows the effect.

### Example 2

### (A) Material and method

### (1) Experimental animals and transplantation of a tumor cell

1.0 X 10⁴ of Lewis lung cancer cells were transplanted to the right femoral subcutaneous portion of 31 6-week old mice (C57BL/6N).

### (2) Administration of a mixture of cyclic polylactic acids

At 2nd day after transplantation, mice were divided into an untreated group and AD-3- (synthesized in the Synthesis Example 2) administered group (0.1 mg-, 0.25 mg-, 0.5 mg- and 1 mg-administered group). The AD-3- administered group was subjected to intra-abdominal administration of the AD-3 suspended in the solvent (propylene glycol) on alternate days, and euthanized at 16th day after administration started.

### (3) Collection of transplanting tissue and measurement

After deep anesthesia, thoracotomy was operated to reflux PBS from a left ventricle. Thereafter, the transplanting tumor tissue was resected, measured for a major axis, minor axis, thickness and weight. In the tumor tissue observed, a region (parenchymal tissue), in which tumor cells aggregated on a surface layer, was found as separated from a spongy tissue, in which a deep portion was filled with blood. Then, after the weight was measured, the spongy tissue was removed, and the weight of the parenchymal tissue on the surface layer was measured.

### (4) Enzyme activity of anaerobic glycolytic system

In the parenchymal tissue (tumor tissue), from which the spongy tissue was removed, and a lung tissue, measurement was conducted for hexokinase (HK), phosphofructokinase (PFK), pyruvic acid kinase (PK) and lactate dehydrogenase (LDH).

### (5) Observation of the colony metastasized to the lung

Following resection of both lungs, metastasized colonies present on individual lobes of the lung were classified into 4 categories, i.e., particularly large, large, medium and small, and numbers thereof were counted.

### (6) Histological observation

A tumor tissue was fixed according to the histological method and, then, embedded in hydrophilic methacrylic resin to make a thin section followed by H-E staining for observation.

### (B) Result and discussion

### (1) Weight of the tumor tissue.

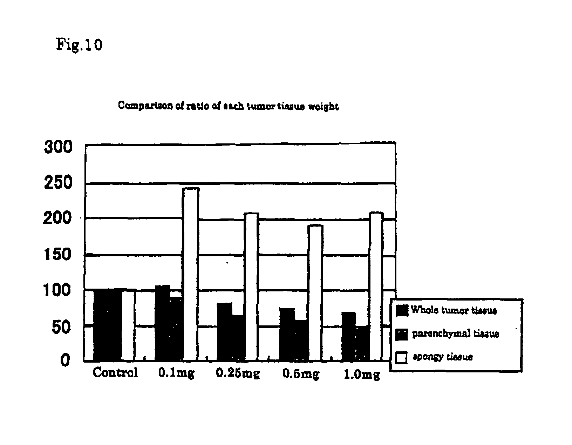

Table 1 shows a weight of the tumor tissue of each group. Fig. 9 shows proportions of the parenchymal tissue to the spongy tissue in each group. Fig. 10 shows weight of a tumor, weight of the parenchymal tissue and weight of the spongy tissue in the drug-administered group, while defining those in the control group as 100.

**Table 1:**

| Comparison of weights of the tumor tissues | | | |
|---|---|---|---|
| | Weight (g) of whole tumor | Weight (g) of parenchymal tissue | Proportion of parenchymal tissue to whole tumor |
| Control group (n=7) | 2.25±0.73 a) | 2.22±0.80 | 0.88 |
| 0.1 mg administered group (n=6) | 2.67±0.70 | 1.94±0.59 | 0.73 |
| 0.25 mg administered group (n=5) | 2.01±0.37 | 1.39±0.30 | 0.69 |
| 0.5 mg administered group (n=6) | 1.85±0.86 | 1.28±0.61 b) | 0.69 |
| 1 mg administered group (n=6) | 1.71±0.24 b) | 1.08±0.15 c) | 0.63 |

| | | | |
|---|---|---|---|
| a): mean±SD b):p<0.05 c):p<0.01 | | | |

As the result, reduction of the weight of the whole tumor tissue and the parenchymal tissue was observed in accordance with increase in the administered amount. The proportion of the parenchymal tissue to the whole tumor tissue also showed a reducing tendency. In addition, in all the AD-3- administered group, the weight of the spongy tissue showed about 200% with a distinct increase compared with the control group.

### (2) Enzyme activity of the anaerobic glycolytic system

For the tumor tissue of each group, an enzyme activity of anaerobic glycolytic system was measured. The result showed a significant decrease in the enzyme activity in 0.1 mg, 0.25 mg and 0.5 mg administered groups (Table 2). However, the measurement of HK, PFK, PK and LDH for the lung tissue showed no significant difference in any administration groups in comparison with the control group.

**Table 2:**

| Comparison of enzyme activities in tumor tissues | | | | |
|---|---|---|---|---|
| | Enzyme activity (mU/ mg protein) | | | |
| | HK | PFK | PK | LDH |
| Control group (n=7) | 97.1±9.8 a) | 122.2±19.9 | 1021±128 | 6548±689 |
| 0.1 mg administered group (n=6) | 77.5±22.8 | 79.7±23.1 b) | 641±204 c) | 5415±1249 |
| 0.25 mg administered group (n=5) | 68.7±14.8 c) | 73.5±7.3 d) | 638±193 c) | 4963±983 c) |
| 0.5 mg administered group (n=6) | 80.1±15.1 b) | 84.1±18.3 b) | 771±90 c) | 5440±543 c) |
| 1 mg administered group (n=6) | 85.2±10.0 | 88.7±18.8 | 904±137 | 5816±723 |

| | | | | |
|---|---|---|---|---|
| a): mean±SD b):p<0.05 c):p<0.01 d):p<0.001 | | | | |

### (3) Histological observation of tumor tissues

The result of microscopic observation of tissue sections showed that the parenchymal tissue had an aggregation of tumor cells including a mitotic view. The spongy tissue was occupied by necrotic cell views and showed infiltration of neutrophils and the like. On the boundary between both tissues, cells were observed containing many swelled vacuoles.

### (4) Conclusion

The result of examination of antitumor action of the AD-3 showed reduction of the transplanting tumor tissue, an increase in the proportion of necrotic cancer cells, and the effect of suppressing the anaerobic glycolytic system.

According to studies conducted by using a conventional material including the CPL, it has been known that carcinogenesis and propagation of the transplanting cancer tissue are suppressed and this phenomenon relates closely to suppression of the anaerobic glycolytic system of cancer cells. The present experiment showed the antitumor effect through administration of a lower dose than that reported so far. Further, the increase in the necrotic cell region (spongy tissue) was observed in the tumor tissue, suggesting an accelerating progress of cell necrosis. This phenomenon was also observed in the 0.25 mg and 0.5 mg administered groups showing no significant difference in sizes and weights of tumors. It is presumed in these groups that the anaerobic glycolytic system is suppressed and the activity of the tumor tissue decreases. Such increase in the spongy tissues in the tumor becomes an important index of the antitumor action of the CPL.

### Industrial Applicability

The present invention makes it possible to provide a novel antitumor agent and also provide food and drink using the same. The antitumor agent according to the present invention can exhibit a sufficient antitumor effect even in a low dose. Further, the mixture of polylactic acids used as an active ingredient in the present invention is a lowly condensed compound of a lactic acid derived from a component of a living body and, therefore, shows a high biocompatibility and a less adverse effect.

## Claims

1. An antitumor agent which comprises a mixture of polylactic acids which contains cyclic polylactic acids having a condensation degree ranging from 3 to 20 as main component.

2. The antitumor agent according to claim 1 wherein the mixture of polylactic acids contains substantially no linear polylactic acid.

3. The antitumor agent according to claim 1 or 2 wherein the mixture of polylactic acids which contains cyclic polylactic acids having a condensation degree ranging from 3 to 20 as main component, is prepared by a method of preparation which comprises:
(i) a first heating step wherein lactic acid is subjected to dehydration and condensation by heating, and condensation and dehydration is carried out under pressure and temperature conditions such that byproduct water is distilled and removed while distillation of lactide is avoided; and
(ii) a second heating step wherein at a temperature higher than that of the first step, the pressure is reduced to 100 mmHg or lower under the pressure and temperature condition such that byproduct water is distilled and removed while distillation of lactide is avoided, and a reaction product of the first heating step is subjected to a further continuing reaction at the reduced pressure under heating so as to produce a dehydrated and condensed product of lactic acid.

4. Food and drink which comprise the antitumor agent according to any of claims 1 to 3.
